# EUROPEAN PATENT APPLICATION

(11) **EP 2 923 719 A1**
(43) Date of publication of application: **30.09.2015**
(21) Application number: 14161349.7
(22) Date of filing: 24.03.2014
(51) Int. Cl.: A61M 16/00

(54) **Medical intelligent ventilation system**

(71) Applicant: Pooyandegan Rah Saadat Co., 1000780715 Tehran (IR); Heyer Medical AG, 56130 Bad Ems (DE)
(72) Inventor: Tari, Abdolreza Yaghoobzadeh, 1000780715 Tehran (IR); Azizzadeh, Hamid, 1000780715 Tehran (IR); Shalbaf, Reza, 1000780715 Tehran (IR); Kullmann, Torsten, 56077 Koblenz (DE); Tietz, Sascha, 56412 Gackenbach (DE); Lanzinger, Oliver, 56412 Boden (DE)
(74) Representative: Schwan Schorer & Partner mbB

(57) **Abstract**

The present invention relates to a intelligent ventilator system comprising a basic ventilator unit (10) with a housing comprising a main controller unit (12), a first display (14), a first controller unit (16) and a pneumatic system (18) for mechanically moving a gas into and out of the lungs of a patient.

According to the invention a S-Viewer (20) is connected to the basic ventilator (10) and comprises a second display (22) and a second controller unit (24).

## Description

The present invention relates to a ventilation intelligent system according to the preamble of claim 1 and to methods for operating such a ventilation system.

### BACKGROUND OF THE INVENTION

A medical ventilator (or simply ventilator in the following) is a machine designed to mechanically move breathable air into and out of the lungs, to provide the mechanism of breathing for a patient who is physically unable to breathe, or breathes insufficiently.

Nowadays, mechanical ventilators as life-support medical device play an important role in critical/intensive care environment in the field of respiratory therapy. The main task of mechanical ventilator is delivering sufficient level of air and oxygen in different breathing modes into a patient lung suffering from respiratory problems. The mechanical ventilation can fully replace a patient's breathing work or assist this procedure partially. Ventilation modes define the pressure, volume and flow characteristics of the delivered breath to a patient.

Known mechanical ventilation systems include a display as a graphical user interface and a main controller unit. The interface display and the main controller unit are used for monitoring all ventilation operational tasks, status, parameters, settings and controlling the therapy procedure. Such one large display unit ventilators suffer from some drawbacks e.g. high power consumption, difficulties in transporting a ventilator, using one display for commanding tasks, monitoring, trend history and getting all types of data such as hospital information system (HIS) and the other status information. Also, in the occurrence of any electronic failure of the display not affecting the ventilation performances, the user is unable to detect whether the device is still operating or not.

It is an object of the present invention to overcome the afore-mentioned drawbacks.

This object is achieved by a ventilator according to claim 1.

### SUMMARY OF THE INVENTION

It is desirable to deliver breath in a way imposing less work of breathing (WOB) on patient. A lung injured patient is not solely surrounded by the ventilator and could be in direct interaction with other medical devices. Considering a patient's current physiological data and ventilator parameters simultaneously results in less damage to a supported patient and less WOB. Therefore, caregivers could choose the best respiratory therapy by the aid of incorporating the ventilator and other medical devices for a lung injured patients.

Intelligent systems have been introduced in different industrial fields especially in the medical industry. In one aspect, the intelligent systems can be used considerably in critical conditions and in the absence of medical staff specialists by providing prompt treatment and/or warning alarms. Since, the respiratory therapy may take for a long time, non-stop attending of medical staff specialists is nearly impossible to provide patient care services. In fact, the existence of an intelligent system capable of collecting and analyzing the patient data reduces the risks of life threatening conditions. In another aspect, choosing the best treatment strategies is so difficult since the physicians encounter enormous patient data from various medical devices. These intelligent systems establish a logic relationship between the different patient real time data to choose the optimum treatment strategy. Thus, the demand for intelligent medical device in the healthcare market has increased with the aim of improving the quality, safety and reliability of delivered patient care.

The invention introduces an intelligent ventilator with a multitasking Smart-Viewer (S-Viewer) unit including processor and a large second display. S-Viewer can gather, display and integrate data from various medical devices, hospital database and the basic ventilator. This ability allows S-Viewer to send modified signal to the basic ventilator and other integrated medical devices in an intellectual manner. These modified signals cause optimization in the performance of the basic ventilator and medical devices and increasing patient safety during respiratory therapy.

The intelligent ventilator is made up of two separate apparatus correlating to each other; the first apparatus is a basic ventilator including a main housing, a small first display unit, the first controller unit, a pneumatic system and a main controller unit. This apparatus is just specified for basic ventilation and representing the ventilator information and tasks in the first display. The other apparatus is a removable network compatible S-Viewer including a large and high resolution second display, the second controller unit and a novel algorithm technique to manage patient vital signs data.

S-Viewer acts as a removable device in connection with the main housing of the basic ventilator. S-Viewer is network and internet compatible so makes communication with HIS, laboratory (LAB), picture archiving and communication system (PACS) and hospital database easily. S-Viewer also could be interconnected with other S-Viewers in different hospital wards via a local network. The user friendly real time software of S-Viewer displays integrated information from basic ventilator and various medical devices into the second display. It also reduces the amount of unnecessary and invaluable tasks within the medical treatment. Consequently, having an access to various data gathered from different medical devices in S-Viewer plays a significant role in managing a patient treatment procedure.

The whole respiratory therapy procedure is optimized due to the coordination between basic ventilator and S-Viewer gathering data from medical devices. Integrated different vital signs data and ventilation information on S-Viewer increase caregivers confidence in choosing the most effective and protective respiratory therapy. This therapy could be exactly specified to an individual patient by considering the patient's real physiological state and vital signs parameters simultaneously.

In present invention, ventilator accuracy and reliability are improved considerably by providing three controller units, one for driving the first display and algorithm techniques regarding respiratory mode functioning in the basic ventilators, the other one in S-Viewer specified for driving the second display, gathering data from all medical devices and analyzing data and the last one acting as a main controller unit responsible for interaction with the pneumatic module. The triple-controllers structure establishes bi-sided communication between each pair of controller units. This controller structure also makes the ventilator running in a way that at least one of the display units always being in communication with the main control unit and provide the user with the ventilator information.

If any single fault or failure in the ventilator hardware or software occurs, the other procedures are substituted to compensate the errors. In addition, the performance complexity of the main control unit is decreased notably and is limited to the interaction with pneumatic system. These strategies are leading us to raise patient safety by decreasing the possibilities of failure in performances of ventilation.

Two strategies are underlined within the intelligent ventilation procedure. In one strategy the respiratory mode parameters of the basic ventilator could be changed in response to received information from the incorporated medical devices. In this case, S-Viewer produces intellectually the modifying signal for basic ventilator in order to change some parameters of ventilation mode in order to optimize the respiratory therapy. In another one, after receiving user inputs via the basic ventilator especially ventilation mode parameters, S-Viewer generates modifying signals for the surrounded medical devices interacting with the patient to optimize their measurement algorithms to improve the respiratory procedure. Therefore, S-Viewer generates signals and commands for modifying and optimizing the basic ventilator and medical device performances in order to enhance the respiratory procedure. It should be noted that the main contribution of this invention is the provision of an intelligent structure and configuration which an intelligent algorithm could be applied in.

### BRIEF DESCRIPTION OF THE DRAWINGS

The present invention is described in more details by the aid of the following figures.
Figure 1 is the block diagram of an intelligent ventilator system.
Figure 2 is the block diagram of an intelligent ventilator system in which the removable S-Viewer is in communication with network and other medical/monitoring devices.
Figure 3 represents the flowchart of data displaying and controlling circuits in the ventilation system.
Figure 4 illustrates an exemplary of the intelligent ventilator screen shot.

### DETAILED DESCRIPTION

In present invention, the embodiments of an intelligent ventilation system including a removable multitasking S-Viewer 20 and basic ventilator 10 with a novel controlling method are described. S-Viewer 20 is implemented with the aim of improving the respiratory procedure by presenting the ventilation and other medical data together, making communication with network and other medical devices and importantly generating signals and commands for modifying and optimizing the basic ventilator 10 and medical devices performances.

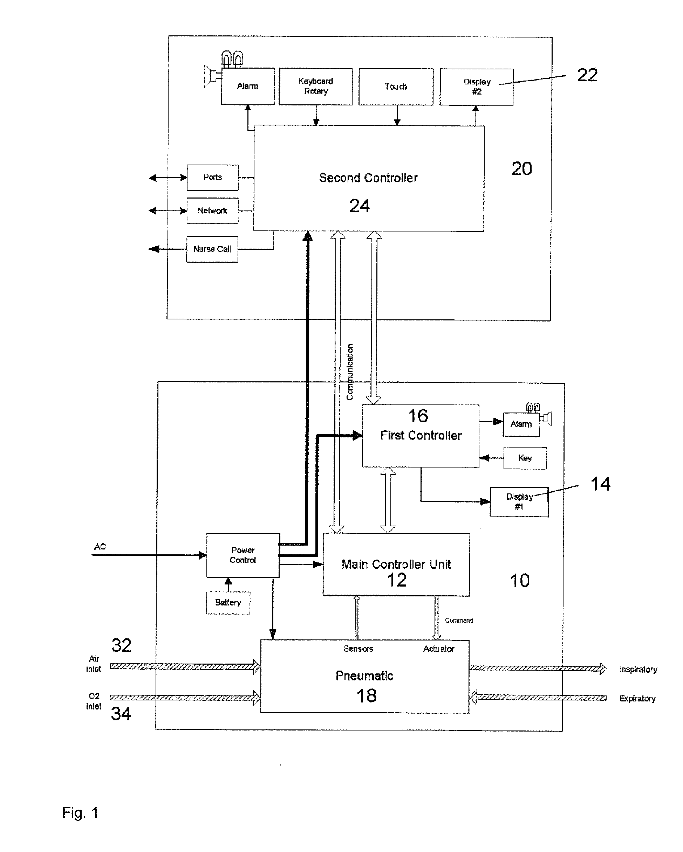

Figure 1 illustrates an intelligent ventilation system configuration. It should be noted that the innovation in this patent is regarding the provision of an intelligent structure and configuration which an intelligent algorithm could be applied in.

The pneumatic system 18 containing inspiratory and expiratory modules is located in the main house of the basic ventilator 10 and coupled with the related limbs for receiving and delivering of the mix gases. In detail, the pneumatic system 18 includes different parts such as two separate inlets (32, 34) for pressurized air and oxygen, oxygen sensor, check valves, safety valve, inhalation and exhalation solenoid, air and oxygen regulators, inhalation and exhalation valves, nebulizer, proportional valves, heat exchanger, flow and pressure sensors, tubing, filters and other components. The pneumatic module which is coupled with a patient via an interface mask receives inputs and commands from ventilator, patient and user for circulating gas through a patient's lung.

A main controller unit 12 located in the main housing of the basic ventilator 10 controls the pneumatic system 18 and subsystems, making communication with first and second display 14, 22 through respective controllers units 16, 24 to receive the user commands, and causes the two display unit 14, 22 presenting the ventilation parameters and other information. This controller transmits algorithm commands created in the first controller unit 16 to the gas delivery system. Also, this controller reads pressure and flow sensors and sending this information to the first and second controller units 16, 24.

The first controller unit 16 runs the first display 14 and controls the first display tasks, analyzes the received commands from user and patient, transmits the user commands including respiratory mode parameters and alarm ranges to the main control unit and converts raw data provided by main control unit and other subsystems to graphical schematics. It also calculates patient respiratory physiological and mechanical parameters as well as ventilator mechanical parameters such as leakage. In addition, a novel algorithm technique is used in this controller 16 for producing of different ventilation modes. Finally, the first controller unit 16 makes a bidirectional communication with the main controller unit 12 and also the second controller unit 24 as well. In the case of any disconnection between the second controller unit 24 and the main control unit 12, the first controller unit 16 fills the gap and acts as a bridge between the main controller unit 12 and the second controller unit 24 for receiving and transmitting commands between these two controllers.

The first display 14 is a small low powered user interface unit placing in the main house of the basic ventilator 10. This first display 14 is used for monitoring the ventilator parameters, settings, configuration, alarms, patient physiological and mechanical parameters, user customization menu, numerical data, changing the current respiratory mode, monitoring the status data of the subsystems such as battery condition, gas sources availability and any other types of status data. In one embodiment, the first display unit 14 may be always on and in another embodiment it may not be always on and turning on in case of second display failure or receiving user command. In the case of being always on, only the status data could be showed in the first display 14. In another situation, all the ventilation operational data could be accessed through this interface as well as second display.

S-Viewer 20 includes a large second display unit 22 and a second controller unit 24.

The second controller unit 24 has a bidirectional relationship with the first controller unit 16 and the main controller unit 12. If any disconnection occurs between the first controller unit 16 and the main controller 12, the commands are transmitted to the main controller unit 12 via the second controller unit 24. This second controller unit 24 also has a two-way connection with network database, HIS, PACS, LAB, vital signs monitors 26, syringe pump 28 and other medical devices 30 through a bidirectional interaction with them. After integrating multiple data from all medical devices 26, 28, 30 and basic ventilator 10, the complicated real time software in the second controller unit 24 employs data fusion technique to improve treatment procedure and breathing pattern. Optimum breathing pattern for patient is gained when both WOB and imposed resistive work are minimized. This controller also interprets and processes raw data obtained from the user, all controllers 12, 16, 24 and all medical devices 26, 28, 30. Incorporating ventilator data with other vital signs parameters help monitoring patient more in detail and optimizing the gas exchange procedure.

The second display 22 is a large, high resolution and high power consumption input and output user interface. The second display 22 shows the ventilator information, the user customization menu and the integrated information from all medical devices 26, 28, 30 in a different graphical format. This large second display 22 presents a well-arranged graphical user interface to monitor and control the ventilator setting, therapy changes, and patient physiological parameters. Presentation format of the ventilation and other sources of data is preselected or user selectable. In one embodiment the second display 22 may be always on and in another embodiment it may be turned on based on the user command.

These controller structures cause the proposed ventilator acting as an intelligent system by generating modifying and controlling signals and suitable alarms for the basic ventilator 10 and connected medical devices 26, 28, 30 in order to improve respiratory therapy. These signals and alarms make performances of all medical devices in the way that more assisting to the respiratory therapy. By combining patient vital signs parameters and basic ventilator 10 data together, intelligent ventilator meets wide range of clinical staff requirements in the field of choosing the optimum treatment strategy for patients suffering from acute lung impaired function. The following five examples are mentioned as some intelligent algorithms which could be applied in the proposed ventilator:
First, for a patient suffering from cardiac problem, applying a specific respiratory therapy mode by ventilator may induce a specific condition to the patient's heart. Therefore in this case, after collecting multiple data from vital signs monitor 26, preferably from ECG electrodes, S-Viewer 20 produce intellectually modifying signal to basic ventilator 10 in order to change parameters of ventilation mode leading to less damage to the patient's heart.
Second, S-Viewer 20 gathers vital signs parameters such as oxygen saturation (Spo2) from a pulse oximetry device to modify ventilation mode parameters. If spo2 value decreases, S-Viewer 20 intellectually makes modifying signal to basic ventilator 10 to increase fraction of delivered oxygen. Also if end tidal CO2 (EtCo2) from capnograph device increases, S-Viewer 20 intellectually makes modifying signal to basic ventilator 10 to increase minute volume.
Third, the ventilation procedure induces periodic changes in invasive blood pressure waveform and pressure oscillation amplitude needed for non-invasive method. These disturbances lead to decreased accuracy of determined mean/systole/diastole arterial pressure. In order to decrease the influences of ventilation on the pressure measurement, S-Viewer 20 produce intellectually modifying signal for pressure measurement devices. Based on this information, the algorithm of these devices is modified within the period of ventilation by ignoring the detected less qualified pressure pulses.
Forth, in the context of depth of anesthesia assessment, high respiratory rate may cause disturbances on the electroencephalogram signal and result in incorrect determination of patient consciousness state. As the ventilation function initiates, the modifying commands are transmitted to the brain function assessment module by S-Viewer 20 to change the cut-off frequency of filter to eliminate the effects of this type of disturbances in the evaluation of patient's anesthetic state.
Fifth, the accuracy of heart rate and arrhythmia detection software in vital signs monitor may be decreased due to the existence disturbance created by the ventilator. Consequently in this case, S-Viewer 20 make automatically functional modifying signal to vital signs monitor with the aim of change the predetermined calculating thresholds and so result in more accuracy in heart rate and arrhythmia detection software.

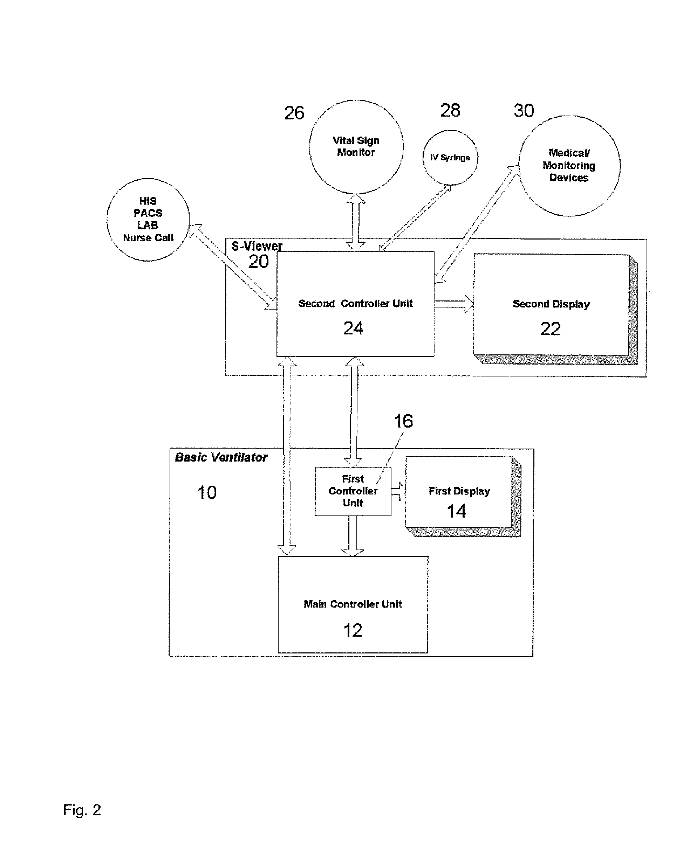

Figure 2 illustrates the intelligent ventilator system including three controller units 12, 16, 24, first display 14 and second display 22. This type of ventilator device not only control the respiratory procedure based on the user input but also display integrated data receiving from all medical devices and hospital database.

One of the main tasks of the main controller unit 12 is controlling pneumatic module 18 and subsystems according to received commands from user. The main controller unit 12 receives the different sensors data and applies command produced by the first and second controller unit 16, 24 to actuators. The controller units function in a way that the main controller unit 12 always is being capable of making communication with the first and second controller units 16, 24.

The second controller unit 24 continually collects vital signs data from all medical devices 26, 28, 30, basic ventilator 10 and hospital database including HIS, PACS and LAB. This controller also transmits the modified and optimized signals to these devices in response to initiation of respiratory therapy. These modified signals could be presented as alarm messages on the basic ventilator 10 and medical devices 26, 28, 30 in order to alert the user about conflicting ventilator mode with vital signs parameters such as electrocardiogram (ECG), Spo2 and non-invasive blood pressure (NIBP). Therefore, this controller prevents high work of breathing done by the patient and some complications such as respiratory muscle fatigue.

The second display 22 is a large and touch input/output user interface. The user commands could be delivered to this second display 22 by the aid of high sensitive touch screen, rotary and keyboard. The first small display 14 is also touch input/output user interface beside the alarm led lamps. The user commands could be also delivered to the first display 14 by the aid of touch screen, keyboard and rotary.

S-Viewer 20 could be interconnected with other S-Viewers in different hospital wards via a local network. This interconnection network between this S-Viewer 20 with different S-Viewers helps caregivers manage different patients and increase patients safety by real-time observing the results of the administered therapy. Therefore, the caregivers are able to gain access to a particular patient data or monitoring a patient medication procedure using this interconnection network between S-Viewers.

The power controller board is connected to three controller units 12, 16, 24, first display 14 and second display 22. The battery status data and type of the power source in use could be accessible through the first display 14 and the second display 22.

In one embodiment the information in the first display 14 and the second display 22 are the same as each other. In another embodiment, the data shown by the two display units 14, 22 are somewhat different. The basic ventilation mode parameters, basic waveforms and patient physiological parameters could be accessible in the first and second display 14, 22 by the user. The historical data, trends, all waveforms, PACS, HIS, LAB and medical devices data could be accessible just in the second display 22 by the user.

If any single fault or failure in the ventilator hardware or software occurs which leads to error in the context of ventilation procedure, the other procedures are substituted. As an example, in case of simultaneous failure in the first and second controller units 16, 24, some important ventilation tasks are carried on via the main controller unit 12. Hence performances of ventilation are still kept safe although nothing is displayed. Weaning a patient from a ventilator after a long-term exposing to respiratory therapy is considered as one of the most challenging issues. An efficient ventilator weaning technique must be utilized to reduce life threatening situations, risks, distress and patient's impression. In present invention, all patients' vital signs are demonstrated beside the ventilator data in S-Viewer 20. Accordingly the integrated features to S-Viewer 20 results in choosing the most appropriate weaning strategy by medical staff, overcoming fighting issue, minimize lung injury and managing the whole procedure of respiratory treatment.

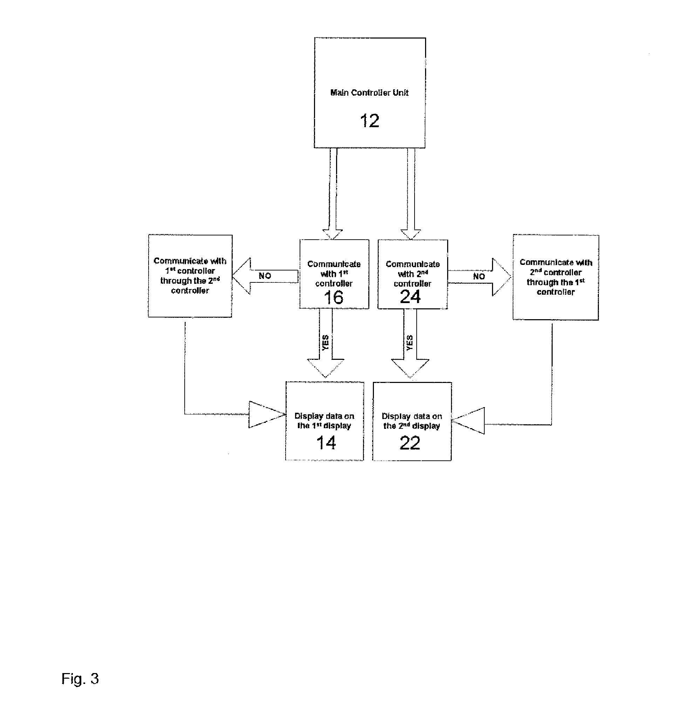

Figure 3 shows the triple controller structure of the intelligent ventilator system in a flowchart form. These structure functions are in a way that the communication between the main controller unit 12, the first controller 16 and the second controller 24 never being disconnected. The main controller unit 12 firstly establishes communication with the first and second controllers 16, 24 in order to display data on each of the display units. In case of any disconnection between the main controller unit 12 and the first controller 16, the main controller 12 establishes connection with the first controller 16 through the second controller 24. That is also true about the communication between the main controller unit 12 and the second controller 24. In other words, if the main controller unit 12 is unable to establish a direct connection with the second controller 24, this connection is carried on through the first controller 16 for presenting data on the second display unit 22. The mentioned controller structure improve safety of the ventilator functioning by making sure that the main controller 12 is always capable of making connection for receiving and transmitting commands of the first and second controller 16, 24.

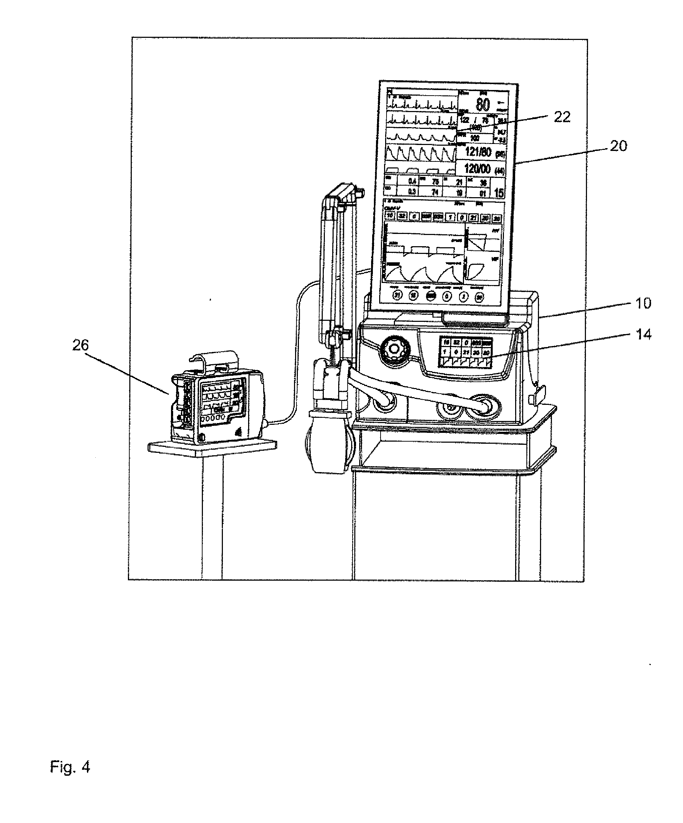

Figure 4 demonstrates an exemplary of the intelligent ventilator configuration. As shown in this figure, this type of ventilator includes a removable S-Viewer 20 connected to the basic ventilator 10 housing. S-Viewer 20 is a network compatible device operating under complicated real-time software. This software collects and process data from vital signs monitor 26 and the basic ventilator 10. S-Viewer 20 shows the ventilator information and the integrated information from vital signs monitor 26 by making two sided connection with these systems. Monitoring patient vital signs parameters in conjunction with the ventilator parameters in one display helps caregivers in evaluating the effectiveness of medical therapy. Network compatible S-Viewer 20 intelligently controls vital signs monitor 26 and the basic ventilator 10 by generating the modifying signals/commands and helping the medical staff to better management of a patient's respiratory procedure.

## Claims

1. An intelligent ventilator system comprising:
a basic ventilator unit (10) with a main house comprising a pneumatic system (18) a main controller unit (12), a first display (14) and a first controller unit (16) for mechanically moving a gas into and out of the lungs of a patient,
**characterized by**
a S-Viewer (20) connectable to the basic ventilator unit (10) and comprising a second display (22) and a second controller unit (24).

2. Ventilator system according to claim 1, **characterized in that** the pneumatic system (18) contains an inspiratory module and an expiratory modules both being located in the basic ventilator housing wherein the pneumatic system is adapted for coupling to a patient via an interface mask or via a tube for tracheal intubation to circulate gas through the patient's lung.

3. Ventilator system according to claim 1 and 2, **characterized in that** the main controller unit (12) is adapted to receive the different sensors data from pneumatic system (18), to control subsystems according to received commands from user and to apply algorithm command produced by the first and second controller unit (16, 24) to actuators.

4. Ventilator system according to claim 1 and 3, **characterized in that** performance complexity of the main control unit (12) is decreased notably and is limited to the interaction with pneumatic system and result in raising patient safety by decreasing the possibilities of main control unit electronic failure.

5. Ventilator system according to anyone of the preceding claims, **characterized in that** the first controller unit (16) runs the first display (14), controls the first display tasks, analyzes the received commands from user and patient, produces different ventilation mode with novel algorithm technique, calculates patient respiratory physiological and mechanical parameters, transmits user commands to the main control unit (12) and converts raw data provided by main control unit (12) and other subsystems to graphical schematics.

6. Ventilator system according to anyone of the preceding claims, **characterized in that** if any single fault or failure in the ventilator hardware or software occurs the other procedures are substituted to compensate the errors and lead to raising patient safety by decreasing the possibilities of failure in performances of ventilation.

7. Ventilator system according to anyone of the preceding claims wherein S-Viewer (20) acts as a removable device including large and high resolution second display (22) and second controller (24) in connection with the main housing of the basic ventilator which displays integrated information from basic ventilator (10) and all medical devices (26, 28, 30) in second display (22).

8. Ventilator system according to claim 1 and 7, **characterized in that** S-Viewer (20) is network and internet compatible so makes communication with HIS, LAB, PACS and hospital database easier and / or the S-Viewer (20) can be interconnected with other S-Viewers in different hospital wards via a local network.

9. Ventilator system according to claim 1 and 7, **characterized in that** the second controller unit (24) embedded in S-Viewer is adapted to run the second display (22), to control the second display tasks, to analyze the received commands from user, to collect data from all medical devices (26, 28, 30), to provide novel real-time algorithm technique to manage variety of medical devices and basic ventilator.

10. Ventilator system according to anyone of the preceding claims, **characterized in that** S-Viewer (20) is adapted to communicate with the basic ventilator (10) and with all medical devices (26, 28, 30) to produce intellectually the adjusting signal for basic ventilator such that a respiratory procedure including parameters of ventilation modes performed by the basic ventilator (10) can be modified based on received information from the incorporated medical devices (26, 28, 30).

11. Method for operating a ventilator system according to claim 10, **characterized in that** the S-Viewer (20) receives oxygen saturation (Spo2) data, preferably from a pulse oximetry device and end tidal CO2 (EtCo2) data from capnograph device, and modifies ventilation mode parameters intellectually such that in case Spo2 value decreases below a predetermined level, S-Viewer (20) increases the fraction of ventilated oxygen, and in case the EtCo2 increases above a second predetermined value, S-Viewer (20) increases the minute volume of ventilation.

12. Method for operating a ventilator system according to claim 10, **characterized in that** in case of the detection of a potential cardiac problem, wherein the detection is based on an analysis of data collected from the all medical devices (26, 28, 30), preferably from ECG electrodes, a specific respiratory therapy including particular ventilation mode parameters is applied by the basic ventilator (10) for reducing damage to the patient's heart, wherein the intellectual analysis is performed by the second controller unit (24).

13. Ventilator system according to anyone of the preceding claims, **characterized in that** S-Viewer (20) is adapted for controlling at least one of the medical devices (26, 28, 30) interacting with the patients such that a procedure including their measurement algorithms performed by the surrounded medical devices (26, 28, 30) can be modified based on received information from the basic ventilator (10) to improve respiratory procedure.

14. Method for operating a ventilator system according to claim 13, **characterized in that**:
S-Viewer (20) is connected to a vital signs monitor and provides functional modifying signals for vital signs monitor, wherein, based on this information, the thresholds for heart rate and arrhythmia detection software performed by the vital signs monitor are modified within the period of ventilating in order to reduce heart rate and arrhythmia detection errors induced by the ventilation.
S-Viewer (20) is connected to a blood pressure measuring device and provides predetermined modifying signals for the blood pressure measurement device, wherein, based on this information, the algorithm of this device is modified within the period of ventilating in order to reduce pressure measuring errors induced by the ventilation.
S-Viewer (20) is connected to a brain function assessment module and provides predetermined modifying signals for the brain function assessment module, wherein, based on this information, the cut-off frequency of filter is modified within the period of ventilating in order to reduce depth of anesthesia assessment errors induced by the ventilation.

15. Method for operating a ventilator system according to anyone of the preceding claims, **characterized in that**:
the main controller unit (12) firstly establishes or tries to establish communication with the first and second controller (16, 24) in order to display data on the first and second display units (14, 22),
in case of any disconnection between the main controller unit (12) and the first controller (16), the main controller (12) establishes connection with the first controller (16) through the second controller (24),
in case of any disconnection between the main controller unit (12) and the second controller (24), the main controller (12) establishes connection with the second controller (24) through the first controller (24),
such that, if the main controller (12) is unable to establish a direct connection with one of the first or second controller (16, 24) or such a direct connection is interrupted, an indirect connection is established through the second or first controller (24, 16) for presenting data on the respective displays (14, 22), wherein, based on this structure, at least one of the display units always being in communication with the main control unit and provide the user with the ventilation information.
